(19) Europäisches Patentamt
European Patent Office
Office européen des brevets

(11) Veröffentlichungsnummer : **0 207 355**
**B1**

(12) **EUROPÄISCHE PATENTSCHRIFT**

(45) Veröffentlichungstag der Patentschrift : **19.10.88**

(51) Int. Cl.[4] : **C 07 D333/38**, C 07 D409/06, A 01 N 43/10, A 01 N 43/84

(21) Anmeldenummer : **86108229.5**

(22) Anmeldetag : **16.06.86**

(54) **Alkoxycarbonyl-substituierte Hydroxythiophen-carbonamide.**

(30) Priorität : **29.06.85 DE 3523313**
**31.01.86 DE 3602889**

(43) Veröffentlichungstag der Anmeldung : **07.01.87 Patentblatt 87/02**

(45) Bekanntmachung des Hinweises auf die Patenterteilung : **19.10.88 Patentblatt 88/42**

(84) Benannte Vertragsstaaten : **CH DE FR GB IT LI**

(56) Entgegenhaltungen :
**EP-A- 0 093 384**
**CHEMICAL ABSTRACTS, Band 101, Nr. 25, 17. Dezember 1984, Columbus, Ohio, USA, L. HUYBRECHTS et al. "The synthesis of 5-carboxamido-4-hydroxy-3-(beta-D-ribofuranosyl) thiophene derivatives analogs of pyrazofurin", p. 807, Zusammenfassung Nr. 230 921j**

(73) Patentinhaber : **BAYER AG**
**Konzernverwaltung RP Patentabteilung**
**D-5090 Leverkusen 1 Bayerwerk (DE)**

(72) Erfinder : **Daum, Werner, Dr.**
**Bärenstrasse 18**
**D-4150 Krefeld (DE)**
Erfinder : **Hänssler, Gerd, Dr.**
**Am Arenzberg 58a**
**D-5090 Leverkusen 3 (DE)**

**EP 0 207 355 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue alkoxycarbonyl-substituierte Hydroxythiophen-carbonamide, ein Verfahren zu ihrer Herstellung und ihre Verwendung als Fungizide und als Zwischenprodukte.

Es ist bereits bekannt, daß 2,5-Bis-(alkoxycarbonyl)-3,4-bis-(acyloxy)-thiophene und 2,5-Bis-(alkoxycarbonyl)-3-alkyl-4-acyloxythiophene fungizide Eigenschaften besitzen (vgl. EP 32 784 und EP 93 384). Hier ist besonders das 2,5-Bis-(isopropoxycarbonyl)-3-methyl-4-(3-methylbenzoyloxy)-thiophen zu nennen, das bekannt ist aus T. Wada et al., Proceedings of the 10th Intern. Congress of Plant Protection, 20.-25.11.1983, Brighton, Vol. 1, 400-407.

Es wurden neue Alkoxycarbonyl-substituierte Hydroxythiophen-carbonamide der allgemeinen Formel (I)

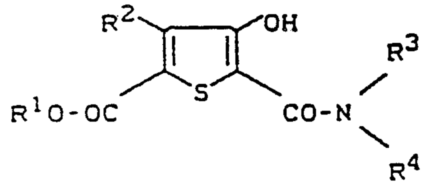

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder gegebenenfalls substituiertes Phenyl steht,

$R^3$ für Alkyl, Alkoxyalkyl, Alkylthioalkyl, Cyanalkyl, Fluoralkyl, Alkenyl, Alkinyl, Cycloalkyl oder Alkoxy steht,

$R^4$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^3$ und $R^4$ gemeinsam mit dem Stickstoffatom für einen gegebenenfalls durch Alkyl substituierten Heterocyclus stehen, welcher in der Alkylenkette Aza-, Oxa- oder Thiaelemente enthalten kann,

gefunden.

Weiterhin wurde gefunden, daß man die neuen Alkoxycarbonyl-substituierten Hydroxythiophen-carbonamide der Formel (I)

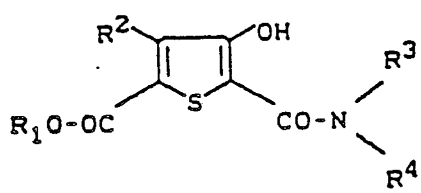

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder gegebenenfalls substituiertes Phenyl steht,

$R^3$ für Alkyl, Alkoxyalkyl, Alkylthioalkyl, Cyanalkyl, Fluoralkyl, Alkenyl, Alkinyl, Cycloalkyl oder Alkoxy steht,

$R^4$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^3$ und $R^4$ gemeinsam mit dem Stickstoffatom für einen gegebenenfalls durch Alkyl substituierten Heterocyclus stehen, welcher in der Alkylenkette Aza-, Oxa- oder Thiaelemente enthalten kann,

erhält, wenn man ein Carbonsäurederivat der Formel (II)

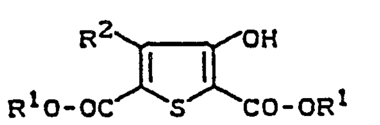

(II)

in welcher $R^1$ und $R^2$ die oben genannten Bedeutungen haben, mit einem Amin der Formel (III)

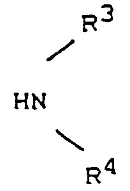

(III)

in der $R^3$ und $R^4$ die vorstehend gennanten Bedeutungen haben, gegebenenfalls in Gegenwart eines Lösungs- oder Verdünnungsmittels und gegebenenfalls in Gegenwart einer tert, organischen Base umsetzt.

Weiterhin wurde gefunden, daß die neuen alkoxycarbonyl-substituierten Hydroxythiophen-carbonamide der Formel (I) fungizide Eigenschaften besitzen und außerdem als Zwischenprodukte zur Synthese aktiver Verbindungen geeignet sind.

Überraschenderweise zeigen die erfindungsgemäßen Alkoxycarbonyl-substituierten Hydroxythiophen-carbonamide der Formel (I) eine bessere fungizide Wirksamkeit als das aus dem Stand der Technik bekannte 2,5-Bis-(isopropoxycarbonyl)-3-methyl-4-(3-methylbenzoyloxy)-thiophen.

Die erfindungsgemäßen Alkoxycarbonyl-substituierten Hydroxythiopen-carbonamide sind durch die Formel (I) allgemein definiert. Bevorzugt sind Verbindungen der Formel I, bei welchen

$R^1$ für Alkyl mit 1 bis 5 Kohlenstoffatomen, für Alkoxyalkyl oder Alkylthioalkyl mit 1 bis 5 Kohlenstoffatomen je Alkylteil, für Fluoralkyl mit je bis zu 5 Fluor- und Kohlenstoffatomen, für Cyanalkyl mit 1 bis 5 Kohlenstoffatomen im Alkylteil, für Alkenyl mit 3 oder 4 Kohlenstoffatomen, für Alkinyl mit 3 bis 5 Kohlenstoffatomen oder für Cycloalkyl mit 4 bis 6 Kohlenstoffatomen steht,

$R^2$ für Alkyl mit 1 bis 4 Kohlenstoffatomen oder für gegebenenfalls ein- bis fünffach, gleich oder verschieden substituiertes Phenyl steht, wobei als Substituenten Alkyl mit 1 bis 4 Kohlenstoffatomen, Alkoxy oder Alkylthio mit je 1 bis 4 Kohlenstoffatomen, Halogen, Nitro, Halogenalkyl, Halogenalkoxy oder Halogenalkylthio mit je 1 bis 4 Kohlenstoffatomen und 1 bis 5 Halogenatomen in Frage kommen. Halogen bedeutet Fluor, Chlor, Brom und Iod, vorzugsweise Fluor und Chlor,

$R^3$ für Alkyl mit 1 bis 5 Kohlenstoffatomen, für Alkoxyalkyl oder Alkylthioalkyl mit jeweils 1 bis 5 Kohlenstoffatomen je Alkylteil, für Cyanalkyl mit 1 bis 5 Kohlenstoffatomen im Alkylteil, für Fluoralkyl mit bis zu 3 Fluoratomen und bis zu 5 Kohlenstoffatomen, für Alkenyl oder Alkinyl mit 3 bis 5 Kohlenstoffatomen, für Cycloalkyl mit 3 bis 6 Kohlenstoffatomen oder für Alkoxy mit 1 bis 5 Kohlenstoffatomen steht,

$R^4$ für Wasserstoff, Alkyl mit 1 bis 5 Kohlenstoffatomen, für Alkoxyalkyl oder Alkylthioalkyl mit jeweils 1 bis 5 kohlenstoffatomen je Alkylteil, für Cyanalkyl mit 1 bis 5 Kohlenstoffatomen im Alkylteil, für Fluoralkyl mit bis zu 3 Fluoratomen und bis zu 5 Kohlenstoffatomen, für Alkenyl oder Alkinyl mit 3 bis 5 Kohlenstoffatomen, für Cycloalkyl mit 3 bis 6 Kohlenstoffatomen steht oder

$R^3$ und $R^4$ zusammen mit dem Stickstoffatom für einem 5-, 6- oder 7-gliedrigen heterocyclischen Ring stehen, der Aza-, Oxa- oder Thiaelemente enthalten und durch Alkylgruppen gegebenenfalls substituiert sein kann.

Besonders bevorzugt sind Verbindungen der Formel (I), bei welchen

$R^1$ für Methyl, Ethyl, n- oder iso-Propyl, 2,2-Dimethylpropyl, 2-Methoxyethyl, 2-Ethoxyethyl, 2-Methylthioethyl, 2-Ethylthioethyl, 2,2,2-Trifluorethyl, Cyanmethyl, Cyanethyl, Allyl, Methallyl, 2-Propinyl, 1,1-Dimethyl-2-propinyl, Cyclopentyl oder Cyclohexyl steht,

$R^2$ für Methyl, Ethyl, n-Propyl, iso-Propyl, n-Butyl, sek.-Butyl, iso-Butyl oder tert.-Butyl, Phenyl oder Halogenphenyl steht,

$R^3$ für Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl, Cyclopropyl, Cyclobutyl, Cyclopentyl, Cyclohexyl, Methoxy, Ethoxy, n-Propyloxy, n-Butyloxy, sek.-Butyloxy oder n-Pentyloxy steht,

$R^4$ für Wasserstoff, Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl, Cyclopropyl, Cyclobutyl, Cyclopentyl, Cyclohexyl steht oder

$R^3$ und $R^4$ zusammen mit dem Stickstoffatom für Pyrrolidin, Piperidin, 2-Methylpiperidin, 3-Methylpiperidin, 4-Methylpiperidin, Hexahydro-1H-azepin, Morpholin, 2,6-Dimethylmorpholin, Thiazolidin, $N^1$-Methylpiperazin oder $N^1$-Propylpiperazin stehen.

Insbesondere seien Verbindungen der Formel (I) genannt, in denen

$R^1$ für Methyl, Ethyl, Isopropyl, n-Propyl, sek.-Butyl, Cyclopentyl oder Cyclohexyl steht,

$R^2$ für Methyl, Ethyl, Isopropyl oder tert.-Butyl oder Phenyl steht,

$R^3$ für Methyl, Butyl oder ω-Cyanpentyl steht,

$R^4$ für Wasserstoff steht oder

$R^3$ und $R^4$ zusammen mit dem Stickstoffatom für Pyrrolidin, Piperidin oder Morpholin stehen.

Verwendet man als Ausgangsverbindungen zur Herstellung der erfindungsgemäßen Verbindungen der Formel (I) beispielsweise 2,5-Bis-(cyclopentyloxycarbonyl)-3-methyl-4-hydroxythiophen und 2-Methoxyethylamin, so kann der Reaktionsverlauf durch das folgende Reaktionsschema wiedergegeben werden :

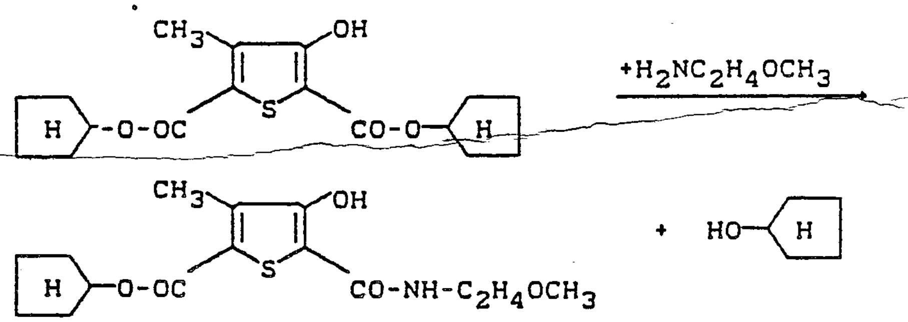


Als Ausgangsstoffe für die Umsetzungen zu den erfindungsgmäßen Verbindungen werden 4-Hydroxythiophen-Derivate benötigt, die durch die Formel (II) allgemein definiert sind. Die Ausgangsverbindungen der Formel (II) sind teilweise bekannt, können aber alle nach generell bekannten Verfahren hergestellt werden, so z. B. aus Thiodiessigsäureestern und 2-Oxocarbonsäureestern unter alkalischen Bedingungen, z. B. unter der Einwirkung von Kaliumtert.-butylat, wobei anschließend an die Kondensation mit einer Säure behandelt wird (vgl. EP 93 384 und DAS 1 020 641). Die Reaktion kann durch das folgende Schema veranschaulicht werden :

$R^2$-CO-CO-OR + $R^1$-O-OC-$CH_2$-S-$CH_2$-CO-O$R^1$

-HOR/-$H_2O$

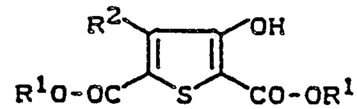

(II)

Als Ausgangsstoffe (II) zu nennen sind im einzelnen : 3-Methyl-4-hydroxy-thiophen-2,5-dicarbonsäure-methyl-, -ethyl-, isopropyl-, -1-methylpropyl-, 2,2-dimethylpropyl-, -cyanmethyl-, -2-cyanethyl-, -1-cyan-1-methyl-ethyl-, -2,2,2-trifluorethyl-, -2-methoxyethyl-, -2-butylthioethyl-, -2-ethyl-thioethyl-, -allyl-, methallyl-, -propargyl-, -1,1-dimethylpropargyl-, -cyclobutyl-, -cyclopentyl- und -cyclohexyl-ester ; 4-Hydroxy-3-ethyl-, -3-propyl-, -3-isopropyl-, -3-butyl-, -3-tert.-butyl- und -3-phenyl-thiophen-2,5-dicarbonsäure-2,2,2-trifluorethylester.

Zur Umsetzung zu den erfindungsgemäßen Verbindungen werden weiterhin primäre oder sekundäre Amine der Formel (III) benötigt, welche literaturbekannt sind, beispielsweise Methylamin, Dimethylamin, Ethylamin, Diethylamin, n-Propylamin, Di-n-propylamin, iso-Propylamin, sek.-Butylamin, iso-Butylamin, n-Butylamin, Di-n-butylamin, Amylamin, N-Methylamylamin, 2-Methoxyethylamin, 2-Ethoxyethylamin, N-Methyl-2-methoxyethylamin, 3-Methoxypropylamin, 3-Butoxypropylamin, 2-Methylthioethylamin, 2-Butylthioethylamin, N-Methyl-3-butylthiopropylamin, 2-Cyanethylamin, 1-Cyan-1-methylethylamin, ω-Cyanpentylamin, 2,2-Difluorethylamin, 2,2,2-Trifluorethylamin, 3,3,3-Trifluorpropylamin, 2,2-Difluorbutylamin 4,4,4-Trifluorbutylamin, 1-Trifluormethylethylamin, Allylamin, Diallylamin, Methallylamin, Propargylamin, N-Methylpropargylamin, Cyclopropylamin, Cyclobutylamin, Cyclopentylamin, Cyclohexylamin, N-Methylcyclohexylamin, N-Butoxyamin, N-Methyl-N-butoxyamin, Pyrrolidin, Piperidin, 2-, 3- oder 4-Methylpiperidin, 2-Ethylpiperidin, Morpholin, 2,6-Dimethylmorpholin, N[1]-Methylpiperazin, N[1]-Propylpiperazin, Thiazolidin und Hexahydro [1H]-azepin.

Als Verdünnungsmittel kommen für das Verfahren alle gegenüber den Reaktionspartnern inerten organischen Lösungsmittel in Frage ; vorzugsweise werden polare Lösungsmittel verwendet. Zu nennen sind hier beispielsweise : Acetonitril, Dimethylacetamid, Dimethylformamid, Dimethylsulfoxid, N-Methylpyrrolidon, Dioxan, Chlorbenzol, Benzonitril, Ethyldiisopropylamin, Triethylamin, Tributylamin, Dimethylcyclohexylamin, Ethyldicyclohexylamin, Dimethylbenzylamin, Pyridin, Picolin und Chinolin oder man verwendet das umzusetzende Amin der Formel (III) als Lösungsmittel.

Die Reaktionstemperaturen und die Reaktionsdauer werden durch die Altivität der Ausgangsprodukte bestimmt. Im allgemeinen arbeitet man etwa zwischen 20 und 180 °C, vorzugsweise zwischen 40 und 150 °C.

Bei der Umsetzung niedrigsiedender Amine kann es von Vorteil sein, die Reaktion unter Druck vorzunehmen.

Je nach Arbeitsbedingungen fallen die erfindungsgemäßen Verbindungen kristallin aus oder sie bleiben im organischen Lösungsmittel gelöst und können nach Abdestillieren überschüssigen Amins, welches aus wirtschaftlichen Gründen möglichst vollständig zurückgewonnen werden soll, und nach Waschen mit Wasser und verdünnter Säure isoliert werden, wobei sie gegebenenfalls durch Zugabe von wenig polaren Lösungsmitteln, wie Cyclohexan, Dibutylether oder Tetrachlorkohlenstoff aus ihren Lösungen abgeschieden werden.

Die erfindungsgemäßen Wirkstoffe weisen eine starke fungizide Wirkung auf und können zur Bekämpfung von unerwünschten Pilzen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt :

Pythium-Arten, wie beispielsweise Pythium ultimum ;
Phytophthora-Arten, wie beispielsweise Phytophthora infestans ;
Pseudoperonospora-Arten, wie beispielsweise Pseudoperonospora humuli oder Pseudoperonospora cubense ;
Plasmopara-Arten, wie beispielsweise Plasmopara viticola ;
Peronospora-Arten, wie beispielsweise Peronospora pisi oder P. brassicae ;
Erysiphe-Arten, wie beispielsweise Erysiphe graminis ;
Sphaerotheca-Arten, wie beispielsweise Sphaerotheca fuliginea ;
Podosphaera-Arten, wie beispielsweise Podosphaera leucotricha ;
Venturia-Arten, wie beispielsweise Venturia inaequalis ;
Pyrenophora-Arten, wie beispielsweise Pyrenophora teres oder P. graminea (Konidienform : Drechslera, Syn : Helminthosporium) ;
Cochliobolus-Arten, wie beispielsweise Cochliobolus sativus (Konidienform : Drechslera, Syn : Helminthosporium) ;
Uromyces-Arten, wie beispielsweise Uromyces appendiculatus ;
Puccinia-Arten, wie beispielsweise Puccinia recondita ;
Tilletia-Arten, wie beispielsweise Tilletia caries ;
Ustilago-Arten, wie beispielsweise Ustilago nuda oder Ustilago avenae ;
Pellicularia-Arten, wie beispielsweise Pellicularia sasakii ;
Pyricularia-Arten, wie beispielsweise Pyricularia oryzae ;
Fusarium-Arten, wie beispielsweise Fusarium culmorum ;
Botrytis-Arten, wie beispielsweise Botrytis cinerea ;
Septoria-Arten, wie beispielsweise Septoria nodorum ;
Leptosphaeria-Arten, wie beispielsweise Leptosphaeria nodorum ;
Cercospora-Arten, wie beispielsweise Cercospora canescens ;
Alternaria-Arten, wie beispielsweise Alternaria brassicae ;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäßen Verbindungen können als Zwischenprodukte zur Herstellung von aktiven Folgeprodukten verwendet werden.

## Herstellungsbeispiele

### Beispiel 1

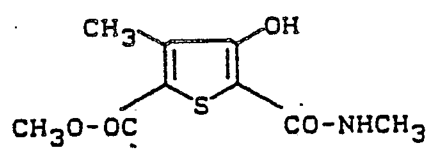

10 g 2,5-Bis-(methoxycarbonyl)-3-methyl-4-hydroxythiophen und 100 ml Benzonitril werden auf 130 °C erhitzt und 15 h lang ein schwacher Strom von Methylamin eingeleitet.

Das Lösungsmittel wird im Vakuum abgezogen. Der Rückstand wird mit Ethylacetat verdünnt, zweimal mit 5 %iger eiskalter Schwefelsäure und zweimal mit Wasser gewaschen. Man trocknet über Natriumsulfat, dampft im Vakuum ein und kristallisiert den Abdampfrückstand aus 20 ml Methanol.

Ausbeute : 1 g 2-Methoxycarbonyl-3-methyl-4-hydroxy-5-methylaminocarbonylthiophen
Schmelzpunkt : 190 °C (Zersetzung) ;
$H^1$-NMR ; 80 MHz, $CDCl_3$ + d-DMSO (δ ppm) :
3H, s 2,38 ($CH_3$) ;
3H, s 3,88 ($CH_3OOC$-) ;
3H, d 2,87 ($CH_3$-NH-).

### Beispiel 2

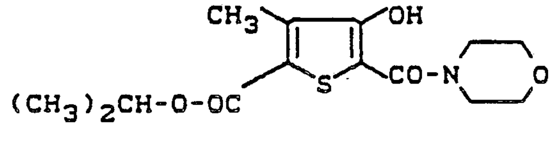

90 g 2,5-Bis-(isopropoxycarbonyl)-3-methyl-4-hydroxythiophen und 450 g Morpholin werden 9 1/2 h auf 124 °C erhitzt. Das überschüssige Morpholin wird im Vakuum abgedampft. Der Abdampfrückstand wird in 600 ml Dichlormethan gelöst, zweimal mit eiskalter, verdünnter Schwefelsäure und einmal mit Wasser gewaschen. Die Lösung wird mit Natriumsulfat getrocknet. Nach Abdampfen des Lösungsmittels wird der Rückstand mit wenig Diisopropylether angerieben, abgetrennt und bei 60 °C/1 mbar getrocknet.

Ausbeute : 78,9 g 2-Isopropoxycarbonyl-3-methyl-4-hydroxy-5-(1-aza-4-oxacyclohex-1-ylcarbonyl)-thiophen mit einem Schmelzpunkt von 123 °C.

Auf gleiche Weise werden die Verbindungen der Formel (I) gewonnen :

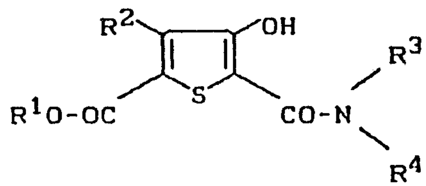

(I)

| Bsp. | $R^1$ | $R^2$ | $R^3$ | $R^4$ | Schmelzpunkt [°C] |
|---|---|---|---|---|---|
| 3 | $H_5C_2$- | $H_3C$- | $-C_2H_4-O-C_2H_4-$ | | 131 |
| 4 | n-$H_7C_3$- | " | " | | 126 |
| 5 | $C_2H_5(CH_3)CH$- | " | " | | 99 |
| 6 | $(CH_3)_2CH$- | " | $-C_5H_{10}-$ | | 86 |
| 7 | " | " | $-C_4H_8-$ | | 134 |
| 8 | " | " | $-(CH_2)_5CN$ | H | 94 |
| 9 | " | " | $-C_6H_{11}$ | H | 124 |
| 10 | $H_3C$- | $(CH_3)_2CH$- | $-C_2H_4-O-C_2H_4-$ | | 115 |

(Fortsetzung)

| Bsp. | $R^1$ | $R^2$ | $R^3$ | $R^4$ | Schmelzpunkt [°C] |
|---|---|---|---|---|---|
| 11 | 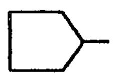 | $H_3C$- | $-C_2H_4-O-C_2H_4-$ | | 123 |
| 12 | $C_2H_5(CH_3)CH-$ | " | $-C_5H_{10}-$ | | viscos |
| 13 | $C_2H_5(CH_3)CH-$ | " | $-C_2H_4OCH_3$ | H | 79 |
| 14 | $H_3C$ | " | " | H | 99 |
| 15 | $H_5C_2-$ | " | $-C_5H_{10}-$ | | 89 |
| 16 | $n-H_7C_3$ | " | " | | 74 |
| 17 | $H_3C$ | 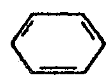 | $-C_2H_4-O-C_2H_4-$ | | 235 |
| 18 | $(H_3C)_2CH-$ | 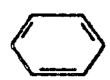 | " | | 151 |

Anwendungsbeispiel

In dem folgenden Anwendungsbeispiel wird die nachfolgend aufgeführte Verbindung als Vergleichssubstanz eingesetzt :

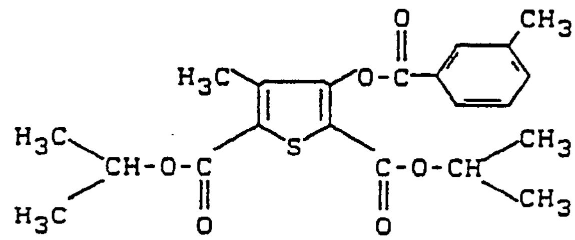

(A)

Pyricularia-Test (Reis)/protektiv

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber der zum Stand der Technik gehören den Verbindung A zeigt in diesem Test z. B. die Verbindung gemäß dem Herstellungsbeispiel 1.

**Patentansprüche**

1. Alkoxycarbonyl-substituierte Hydroxythiophencarbonamide der allgemeinen Formel (I),

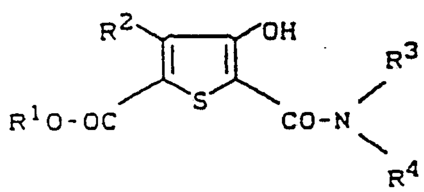

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder gegebenenfalls substituiertes Phenyl steht,

$R^3$ für Alkyl, Alkoxyalkyl, Alkylthioalkyl, Cyanalkyl, Fluoralkyl, Alkenyl, Alkinyl, Cycloalkyl oder Alkoxy steht,

$R^4$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^3$ und $R^4$ gemeinsam mit dem Stickstoffatom für einen gegebenenfalls durch Alkyl substituierten Heterocyclus stehen, welcher in der Alkylenkette Aza-, Oxa- oder Thiaelemente enthalten kann.

2. Alkoxycarbonyl-substituierte Hydroxythiophen-carbonamide der Formel (I) gemäß Anspruch 1, bei welchen

$R^1$ für Alkyl mit 1 bis 5 Kohlenstoffatomen, für Alkoxyalkyl oder Alkylthioalkyl mit 1 bis 5 Kohlenstoffatomen je Alkylteil, für Fluoralkyl mit je bis zu 5 Fluor- und Kohlenstoffatomen, für Cyanalkyl mit 1 bis 5 Kohlenstoffatomen im Alkylteil, für Alkenyl mit 3 oder 4 Kohlenstoffatomen, für Alkinyl mit 3 bis 5 Kohlenstoffatomen oder für Cycloalkyl mit 4 bis 6 Kohlenstoffatomen steht,

$R^2$ für Alkyl mit 1 bis 4 Kohlenstoffatomen oder für gegebenenfalls ein- bis fünffach, gleich oder verschieden durch Alkyl mit 1 bis 4 Kohlenstoffatomen, Alkoxy oder Alkylthio mit je 1 bis 4 Kohlenstoffatomen, Halogen, Nitro, Halogenalkyl, Halogenalkoxy oder Halogenalkylthio mit je 1 bis 4 Kohlenstoffatomen und 1 bis 5 Halogenatomen substituiertes Phenyl steht,

$R^3$ für Alkyl mit 1 bis 5 Kohlenstoffatomen, für Alkoxyalkyl oder Alkylthioalkyl mit jeweils 1 bis 5 Kohlenstoffatomen je Alkylteil, für Cyanalkyl mit 1 bis 5 Kohlenstoffatomen in Alkylteil, für Fluoralkyl mit bis zu 3 Fluoratomen und bis zu 5 Kohlenstoffatomen, für Alkenyl oder Alkinyl mit 3 bis 5 Kohlenstoffatomen, für Cycloalkyl mit 3 bis 6 Kohlenstoffatomen oder für Alkoxy mit 1 bis 5 Kohlenstoffatomen steht,

$R^4$ für Wasserstoff, Alkyl mit 1 bis 5 Kohlenstoffatomen, für Alkoxyalkyl oder Alkylthioalkyl mit jeweils 1 bis 5 Kohlenstoffatomen je Alkylteil, für Cyanalkyl mit 1 bis 5 Kohlenstoffatomen im Alkylteil, für Fluoralkyl mit bis zu 3 Fluoratomen und bis zu 5 Kohlenstoffatomen, für Alkenyl oder Alkinyl mit 3 bis 5 Kohlenstoffatomen, für Cycloalkyl mit 3 bis 6 Kohlenstoffatomen steht oder

$R^3$ und $R^4$ zusammen mit dem Stickstoffatom für einen 5-, 6- oder 7-gliedrigen heterocyclischen Ring stehen, der Aza-, Oxa- oder Thiaelemente enthalten kann und durch Alkylgruppen gegebenenfalls substituiert sein kann.

3. Alkoxycarbonyl-substituierte Hydroxythiophen-carbonamide der Formel (I) gemäß Anspruch 1, in welchen

$R^1$ für Methyl, Ethyl, n- oder iso-Propyl, 2,2-Dimethylpropyl, 2-Methoxyethyl, 2-Ethoxyethyl, 2-Methylthioethyl, 2-Ethylthioethyl, 2,2,2-Trifluorethyl, Cyanmethyl, Cyanethyl, Allyl, Methallyl, 2-Propinyl, 1,1-Dimethyl-2-propinyl, Cyclopentyl oder Cyclohexyl steht,

$R^2$ für Methyl, Ethyl, n-Propyl, iso-Propyl, n-Butyl, sek.-Butyl, iso-Butyl, tert.-Butyl, Phenyl oder Halogenphenyl steht,

$R^3$ für Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl, Cyclopropyl, Cyclobutyl, Cyclopentyl, Cyclohexyl, Methoxy, Ethoxy, n-Propyloxy, n-Butyloxy, sek.-Butyloxy oder n-Pentyloxy steht,

$R^4$ für Wasserstoff, Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl, Cyclopropyl, Cyclobutyl, Cyclopentyl oder Cyclohexyl steht oder

$R^3$ und $R^4$ zusammen mit dem Stickstoffatom für Pyrrolidin, Piperidin, 2-Methylpiperidin, 3-Methylpiperidin, 4-Methylpiperidin, Hexahydro-1H-azepin, Morpholin, 2,6-Dimethylmorpholin, Thiazolidin, $N^1$-Methylpiperazin oder $N^1$-Propylpiperazin stehen.

4. Alkoxycarbonyl-substituierte Hydroxythiophen-carbonamide der formel (I) gemäß Anspruch 1, in welchen

$R^1$ für Methyl, Ethyl, Isopropyl, n-Propyl, sek.-Butyl, Cyclopentyl oder Cyclohexyl steht,

$R^2$ für Methyl, Ethyl, Isopropyl, tert.-Butyl oder Phenyl steht,

$R^3$ für Methyl, Butyl oder ω-Cyanpentyl steht,

$R^4$ für Wasserstoff steht oder

$R^3$ und $R^4$ zusammen mit dem Stickstoffatom für Pyrrolidin, Piperidin oder Morpholin stehen.

5. Verfahren zur Herstellung von Alkoxycarbonyl-substituierten Hydroxy-thiophen-carbonamiden der Formel (I)

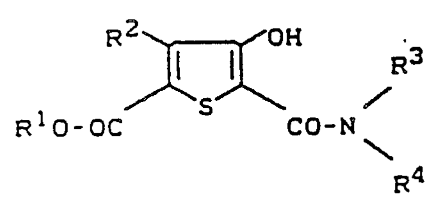

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder für gegebenenfalls substituiertes Phenyl steht,

$R^3$ für Alkyl, Alkoxyalkyl, Alkylthioalkyl, Cyanalkyl, Fluoralkyl, Alkenyl, Alkinyl, Cycloalkyl oder Alkoxy steht,

$R^4$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^3$ und $R^4$ gemeinsam mit dem Stickstoffatom für einen gegebenenfalls durch Alkyl substituierten Heterocyclus stehen, welcher in der Alkylenkette Aza-, Oxa- oder Thiaelemente enthalten kann, dadurch gekennzeichnet, daß man Carbonsäurederivate der Formel (II)

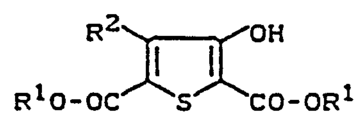

(II)

in welcher $R^1$ und $R^2$ die oben genannten Bedeutungen haben, mit einem Amin der Formel (III)

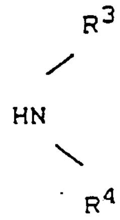

(III)

in der $R^3$ und $R^4$ die vorstehend genannten Bedeutungen haben, gegebenenfalls in Gegenwart eines Lösungs- oder Verdünnungsmittels und gegebenenfalls in Gegenwart einer tert. organischen Base umsetzt.

6. Fungizide Mittel, gekennzeichnet durch einen Gehalt an mindestens einem Alkoxycarbonylsubstituiertes Hydroxythiophen-carbonamid der Formel (I) gemäß den Ansprüchen 1 bis 5.

7. Verfahren zur Bekämpfung von Pilzen ausgenommen die vom Gesetz her nicht schutzfähigen Verfahren, dadurch gekennzeichnet, daß man Alkoxycarbonyl-substituierte Hydroxythiophen-carbonamide der Formel (I) gemäß den Ansprüchen 1 bis 5 auf Pilze und/oder ihren Lebensraum einwirken läßt.

8. Verwendung von Alkoxycarbonyl-substituierten Hydroxythiophen-carbonamiden der Formel (I) gemäß den Ansprüchen 1 bis 5 zur Bekämpfung von Pilzen ausgenommen die vom Gesetz her nicht schutzfähigen Verwendungen.

9. Verfahren zur Herstellung von fungiziden Mitteln, dadurch gekennzeichnet, daß man Alkoxycarbonyl-substituierte Hydroxythiophen-carbonamide der Formel (I) gemäß den Ansprüchen 1 bis 5 mit Streckmitteln vermischt.

**Claims**

1. Alkoxycarbonyl substituted hydroxythiophene-carboxamides of the general formula (I)

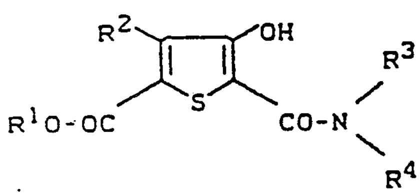

(I)

in which

$R^1$ represents alkyl, alkoxyalkyl, alkylthioalkyl, fluoroalkyl, cyanoalkyl, alkenyl or alkinyl or represents cycloalkyl,

$R^2$ represents alkyl or optionally substituted phenyl,

$R^3$ represents alkyl, alkoxyalkyl, alkylthioalkyl, cyanoalkyl, fluoroalkyl, alkenyl, alkinyl, cycloalkyl or alkoxy and

$R^4$ represents hydrogen, alkyl, alkoxyalkyl, alkylthioalkyl, fluoroalkyl, cyanoalkyl, alkenyl, alkinyl or cycloalkyl,

or

$R^3$ and $R^4$, together with the nitrogen atom, represent a heterocyclic radical which is optionally substituted by alkyl and which can contain aza, oxa or thia elements in the alkylene chain.

2. Alkoxycarbonyl-substituted hydroxythiophene-carboxamides of the formula (I) according to Claim 1,

in which

$R^1$ represents alkyl with 1 to 5 carbon atoms, or represents alkoxyalkyl or alkylthioalkyl with 1 to 5 carbon atoms per alkyl part, or represents fluoroalkyl with in each case up to 5 fluorine and carbon atoms, or represents cyanoalkyl with 1 to 5 carbon atoms in the alkyl part, or represents alkenyl with 3 or 4 carbon atoms, or represents alkinyl with 3 to 5 carbon atoms, or represents cycloalkyl with 4 to 6 carbon atoms,

$R^2$ represents alkyl with 1 to 4 carbon atoms, or represents phenyl which is optionally monosubstituted to pentasubstituted by identical or different substituents, suitable substituents being alkyl with 1 to 4 carbon atoms, alkoxy or alkylthio with in each case 1 to 4 carbon atoms, halogen, nitro, halogenoalkyl, halogenoalkoxy or halogenoalkylthio with each 1 to 4 carbon atoms and 1 to 5 halogen atoms,

$R^3$ represents alkyl with 1 to 5 carbon atoms, or represents alkoxyalkyl or alkylthioalkyl with in each case 1 to 5 carbon atoms per alkyl part, or represents cyanoalkyl with 1 to 5 carbon atoms in the alkyl part, or represents fluoroalkyl with up to 3 fluorine atoms and up to 5 carbon atoms, or represents alkenyl or alkinyl with 3 to 5 carbon atoms, or represents cycloalkyl with 3 to 6 carbon atoms, or represents alkoxy with 1 to 5 carbon atoms and

$R^4$ represents hydrogen or alkyl with 1 to 5 carbon atoms, or represents alkoxyalkyl or alkylthioalkyl with in each case 1 to 5 carbon atoms per alkyl part, or represents cyanoalkyl with 1 to 5 carbon atoms in the alkyl part, or represents fluoroalkyl with up to 3 fluorine atoms and up to 5 carbon atoms, or represents alkenyl or alkinyl with 3 to 5 carbon atoms, or represents cycloalkyl with 3 to 6 carbon atoms, or

$R^3$ and $R^4$, together with the nitrogen atom, represent a 5-, 6- 7-membered heterocyclic ring which contain aza, oxa or thia elements and is optionally substituted by alkyl groups.

3. Alkoxycarbonyl-substituted hydroxythiophene-carboxamides of the formula (I) according to Claim 1,

in which

$R^1$ represents methyl, ethyl, n- or iso-propyl, 2,2-dimethylpropyl, 2-methoxyethyl, 2-ethoxyethyl, 2-methylthioethyl, 2-ethylthioethyl, 2,2,2-trifluoroethyl, cyanomethyl, cyanoethyl, allyl, methallyl, 2-propinyl, 1,1-dimethyl-2-propinyl, cyclopentyl or cyclohexyl,

$R^2$ represents methyl, ethyl, n-propyl, iso-propyl, n-butyl, sec.-butyl, iso-butyl, tert.-butyl, phenyl or halogenophenyl,

$R^3$ represents methyl, ethyl, n- or iso-propyl, 2,2-dimethylpropyl, 2-methoxyethyl, 2-ethoxyethyl, 2-propoxyethyl, 3-methoxypropyl, 3-ethoxypropyl, 3-butoxypropyl, 2-methylthioethyl, 2-ethylthioethyl, 2,2,2-trifluoroethyl, 2-cyanoethyl, 1-methyl-1-cyanoethyl, ω-cyanopentyl, allyl, methallyl, 2-propinyl, 1,1-dimethyl-2-propinyl, cyclopropyl, cyclobutyl, cyclopentyl, cyclohexyl, methoxy, ethoxy, n-propyloxy, n-butyloxy, sec.-butyloxy or n-pentyloxy, and

$R^4$ represents hydrogen, methyl, ethyl, n- or isopropyl, 2,2-dimethylpropyl, 2-methoxyethyl, 2-ethoxyethyl, 2-propoxyethyl, 3-methoxypropyl, 3-ethoxypropyl, 3-butoxypropyl, 2-methylthioethyl, 2-ethylthioethyl, 2,2,2-trifluoroethyl, 2-cyanoethyl, 1-methyl-1-cyanoethyl, ω-cyanopentyl, allyl, methallyl, 2-propinyl, 1,1-dimethyl-2-propinyl, cyclopropyl, cyclobutyl, cyclopentyl or cyclohexyl, or

$R^3$ and $R^4$, together with the nitrogen atom, represent pyrrolidine, piperidine, 2-methylpiperidine, 3-methylpiperidine, 4-methylpiperidine, hexahydro-1H-azepine, morpholine, 2,6-dimethylmorpholine, thiazolidine, $N^1$-methylpiperazine or $N^1$-propylpiperazine.

4. Alkoxycarbonyl substituted hydroxythiophene-carboxamides of the formula (I) according to Claim 1,

in which

$R^1$ represents methyl, ethyl, isopropyl, n-propyl, sec.-butyl, cyclopentyl or cyclohexyl,

$R^2$ represents methyl, ethyl, isopropyl, tert.-butyl or phenyl,

$R^3$ represents methyl, butyl or ω-cyanopentyl and

$R^4$ represents hydrogen, or

$R^3$ and $R^4$, together with the nitrogen atom, represent pyrrolidine, piperidine or morpholine.

5. Process for the preparation of alkoxycarbonyl-substituted hydroxy-thiophene-carboxamides of the formula (I)

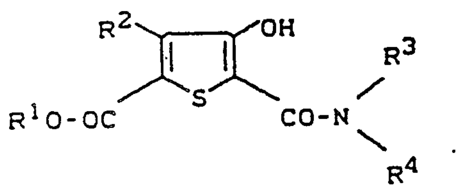

(I)

in which

$R^1$ represents alkyl, alkoxyalkyl, alkylthioalkyl, fluoroalkyl, cyanoalkyl, alkenyl or alkinyl or represents cycloalkyl,

$R^2$ represents alkyl or optionally substituted phenyl,

$R^3$ represents alkyl, alkoxyalkyl, alkylthioalkyl, cyanoalkyl, fluoroalkyl, alkenyl, alkinyl, cycloalkyl or alkoxy and

$R^4$ represents hydrogen, alkyl, alkoxyalkyl, alkylthioalkyl, fluoroalkyl, cyanoalkyl, alkenyl, alkinyl or cycloalkyl,

or

$R^3$ and $R^4$, together with the nitrogen atom, represent a heterocyclic radical which is optionally substituted by alkyl and which can contain aza, oxa or thia elements in the alkylene chain, characterized in that carboxylic acid derivatives of the formula (II)

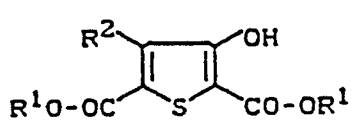

(II)

in which $R^1$ and $R^2$ have the abovementioned meanings, are reacted with an amine of the formula (III)

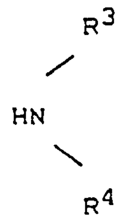

(III)

in which $R^3$ and $R^4$ have the abovementioned meanings, if appropriate in the presence of a solvent or diluent and if appropriate in the presence of a tertiary organic base.

6. Fungicidal agents, characterized in that they contain at least one alkoxycarbonyl-substituted hydroxythiophene-carboxamide of the formula (I) according to Claims 1 to 5.

7. Method of combating fungi with the exception of methods which, by law, are not capable of protection, characterized in that alkoxycarbonyl-substituted hydroxythiophene-carboxamides of the formula (I) according to Claims 1 to 5 are allowed to act on fungi and/or their environment.

8. Use of alkoxycarbonyl-substituted hydroxythiophene-carboxamides of the formula (I) according to Claims 1 to 5 for combating fungi, with the exception of uses which, by law, are not capable of protection.

9. Process for the preparation of fungicidal agents, characterized in that alkoxycarbonyl-substituted hydroxythiophene-carboxamides of the formula (I) according to Claims 1 to 5 are mixed with extenders.

**Revendications**

1. Hydroxythiophène-carboxamides à substituants alkoxycarbonyle de formule générale (I)

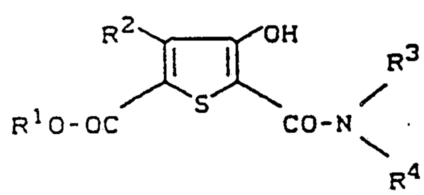

(I)

dans laquelle

$R^1$ représente un groupe alkyle, alkoxyalkyle, alkylthioalkyle, fluoralkyle, cyanalkyle, alcényle, alcynyle ou cycloalkyle,

$R^2$ est un groupe alkyle ou un groupe phényle éventuellement substitué,

$R^3$ est un groupe alkyle, alkoxyalkyle, alkylthioalkyle, cyanalkyle, fluoralkyle, alcényle, alcynyle, cycloalkyle ou alkoxy,

$R^4$ représente l'hydrogène, un groupe alkyle, alkoxyalkyle, alkylthioalkyle, fluoralkyle, cyanalkyle, alcényle, alcynyle ou cycloalkyle,

ou bien

$R^3$ et $R^4$ forment, conjointement avec l'atome d'azote, un hétérocycle éventuellement substitué par un radical alkyle, qui peut contenir dans la chaîne alkylénique des éléments aza, oxa ou thia.

2. Hydroxythiophène-carboxamides à substituants alkoxycarbonyle, de formule (I) suivant la revendication 1, dans lesquels

$R^1$ est un groupe alkyle de 1 à 5 atomes de carbone, un groupe alkoxyalkyle ou alkylthioalkyle ayant 1 à 5 atomes de carbone par radical alkyle, un groupe fluoralkyle ayant jusqu'à 5 atomes de fluor et de carbone, un groupe cyanalkyle ayant 1 à 5 atomes de carbone dans le radical alkyle, un groupe alcényle de 3 ou 4 atomes de carbone, un groupe alcynyle de 3 à 5 atomes de carbone ou un groupe cycloalkyle de 4 à 6 atomes de carbone,

$R^2$ est un groupe alkyle de 1 à 4 atomes de carbone ou un groupe phényle portant éventuellement 1 à 5 substituants, identiques ou différents, alkyle ayant 1 à 4 atomes de carbone, alkoxy ou alkylthio ayant chacun 1 à 4 atomes de carbone, halogéno, nitro, halogénalkyle, halogénalkoxy ou halogénylalkylthio ayant chacun 1 à 4 atomes de carbone et 1 à 5 atomes d'halogènes,

$R^3$ est un groupe alkyle de 1 à 5 atomes de carbone, un groupe alkoxyalkyle ou alkylthioalkyle ayant 1 à 5 atomes de carbone par radical alkyle, un groupe cyanalkyle de 1 à 5 atomes de carbone dans le radical alkyle, un groupe fluoralkyle ayant jusqu'à 3 atomes de fluor et jusqu'à 5 atomes de carbone, un groupe alcényle ou alcynyle ayant 3 à 5 atomes de carbone, un groupe cycloalkyle de 3 à 6 atomes de carbone ou un groupe alkoxy de 1 à 5 atomes de carbone,

$R^4$ représente l'hydrogène, un groupe alkyle de 1 à 5 atomes de carbone, alkoxyalkyle ou alkylthioalkyle ayant chacun 1 à 5 atomes de carbone par radical alkyle, un groupe cyanalkyle de 1 à 5 atomes de carbone dans le radical alkyle, un groupe fluoralkyle ayant jusqu'à 3 atomes de fluor et jusqu'à 5 atomes de carbone, un groupe alcényle ou alcynyle ayant 3 à 5 atomes de carbone, un groupe cycloalkyle de 3 à 6 atomes de carbone, ou bien

$R^3$ et $R^4$ forment conjointement avec l'atome d'azote un noyau hétérocyclique pentagonal, hexagonal ou heptagonal qui peut comporter des éléments aza, oxa ou thia et qui peut être substitué éventuellement par des groupes alkyle.

3. Hydroxythiophène-carboxamides à substituants alkoxycarbonyle de formule (I) suivant la revendication 1, dans lesquels

$R^1$ est un groupe méthyle, éthyle, n-propyle ou isopropyle, 2,2-diméthylpropyle, 2-méthoxyéthyle, 2-éthoxyéthyle, 2-méthylthioéthyle, 2-éthylthioéthyle, 2,2,2-trifluoréthyle, cyanométhyle, cyanéthyle, allyle, méthallyle, 2-propynyle, 1,1-diméthyl-2-propynyle, cyclopentyle ou cyclohexyle,

$R^2$ est un groupe méthyle, éthyle, n-propyle, isopropyle, n-butyle, sec.-butyle, isobutyle, tertio-butyle, phényle ou halogénophényle,

$R^3$ est un groupe méthyle, éthyle, n-propyle ou isopropyle, 2,2-diméthylpropyle, 2-méthoxyéthyle, 2-éthoxyéthyle, 2-propoxyéthyle, 3-méthoxypropyle, 3-éthoxypropyle, 3-butoxypropyle, 2-méthylthioéthyle, 2-éthylthioéthyle, 2,2,2-trifluoréthyle, 2-cyanéthyle, 1-méthyl-1-cyanéthyle, ω-cyanopentyle, allyle, méthallyle, 2-propynyle, 1,1-diméthyl-2-propynyle, cyclopropyle, cyclobutyle, cyclopentyle, cyclohexyle, méthoxy, éthoxy, n-propyloxy, n-butyloxy, sec.-butyloxy ou n-pentyloxy,

$R^4$ représente l'hydrogène, un groupe méthyle, éthyle, n-propyle ou isopropyle, 2,2-diméthylpropyle, 2-méthoxyéthyle, 2-éthoxyéthyle, 2-propoxyéthyle, 3-méthoxypropyle, 3-éthoxypropyle, 3-butoxypropyle, 2-méthylthioéthyle, 2-éthylthioéthyle, 2,2,2-trifluoréthyle, 2-cyanéthyle, 1-méthyl-1-cyanéthyle, ω-cyanopentyle, allyle, méthallyle, 2-propynyle, 1,1-diméthyl-2-propynyle, cyclopropyle, cyclobutyle, cyclopentyle ou cyclohexyle, ou bien

$R^3$ et $R^4$ forment conjointement avec l'atome d'azote un noyau de pyrrolidine, pipéridine, 2-méthylpipéridine, 3-méthylpipéridine, 4-méthylpipéridine, hexahydro-1H-azépine, morpholine, 2,6-diméthylmorpholine, thiazolidine, $N^1$-méthylpipérazine ou $N^1$-propylpipérazine.

4. Hydroxythiophène-carboxamides à substituants alkoxycarbonyle de formule (I) suivant la revendication 1, dans lesquels

$R^1$ représente un groupe méthyle, éthyle, isopropyle, n-propyle, sec.-butyle, cyclopentyle ou cyclohexyle,

$R^2$ est un groupe méthyle, éthyle, isopropyle, tertio-butyle, ou phényle,

$R^3$ est un groupe méthyle, butyle ou ω-cyanopentyle,

$R^4$ représente l'hydrogène,

ou bien

$R^3$ et $R^4$ forment, conjointement avec l'atome d'azote, un noyau de pyrrolidine, pipéridine ou morpholine.

5. Procédé de production d'hydroxythiophènecarboxamides à substituants alkoxycarbonyle, de formule (I)

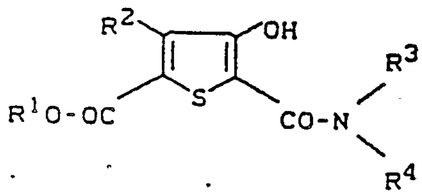

(I)

dans laquelle

$R^1$ représente un groupe alkyle, alkoxylalkyle, alkylthioalkyle, fluoralkyle, cyanalkyle, alcényle, alcynyle, ou cycloalkyle,

$R^2$ est un groupe alkyle ou un groupe phényle éventuellement substitué,

$R^3$ est un groupe alkyle, alkoxyalkyle, alkylthioalkyle, cyanalkyle, fluoralkyle, alcényle, alcynyle, cycloalkyle ou alkoxy,

$R^4$ représente l'hydrogène, un groupe alkyle, alkoxyalkyle, alkylthioalkyle, fluoralkyle, cyanalkyle, alcényle, alcynyle ou cycloalkyle,

ou bien

$R^3$ et $R^4$ forment, conjointement avec l'atome d'azote, un hétéroxycle éventuellement substitué par un radical alkyle, qui peut contenir dans la chaîne alkylénique des éléments aza, oxa ou thia.

caractérisé en ce qu'on fait réagir des dérivés d'acides carboxyliques de formule (II)

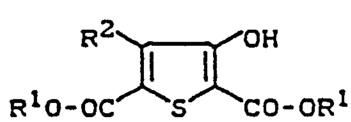

(II)

dans laquelle $R^1$ et $R^2$ ont les définitions données ci-dessus, avec une amine de formule (III)

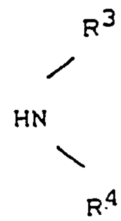

(III)

dans laquelle $R^3$ et $R^4$ ont les définitions données ci-dessus, éventuellement en présence d'un solvant ou d'un diluant et le cas échéant en présence d'une base organique tertiaire.

6. Compositions fongicides, caractérisées par une teneur en au moins un hydroxythiophène-carboxamide à substituant alkoxycarbonyle de formule (I) suivant les revendications 1 à 5.

7. Procédé pour combattre des champignons, à l'exclusion des procédés non susceptibles d'être protégés par la loi, caractérisé en ce qu'on fait agir des hydroxythiophène-carboxamides à substituants alkoxycarbonyle de formule (I) suivant les revendications 1 à 5 sur des champignons et/ou sur leur milieu.

8. Utilisation d'hydroxythiophène-carboxamides à substituants alkoxycarbonyle de formule (I) suivant les revendications 1 à 5 pour combattre des champignons, à l'exclusion des utilisations non susceptibles d'être protégées par la loi.

9. Procédé de préparation de compositions fongicides, caractérisé en ce qu'on mélange des hydroxythiophène-carboxamides à substituants alkoxycarbonyle de formule (I) suivant les revendications 1 à 5 avec des diluants.